# EUROPEAN PATENT APPLICATION

(11) **EP 4 505 988 A1**
(43) Date of publication of application: **12.02.2025**
(21) Application number: 23784712.4
(22) Date of filing: 31.03.2023
(51) Int. Cl.: A61G 12/00, B25J 13/08, G06Q 50/22

(54) **NURSING-CARE ROBOT SYSTEM AND NURSING-CARE ROBOT CONTROL DEVICE**

(30) Priority: 08.04.2022 JP 2022064661
(71) Applicant: Itoki Corporation, Osaka-shi, Osaka 541-0047 (JP)
(72) Inventor: OSAWA Kouichi, Osaka-shi, Osaka 541-0047 (JP); YOSHIOKA Fumihiro, Osaka-shi, Osaka 541-0047 (JP); EHARA Kuniaki, Osaka-shi, Osaka 541-0047 (JP); NISHIMOTO Shogo, Osaka-shi, Osaka 541-0047 (JP)
(74) Representative: Kilian Kilian & Partner mbB
(86) International application number: PCT/JP2023/013555
(87) International publication number: WO 2023/195425

(57) **Abstract**

An object is to be able to provide a nursing-care robot system which can perform an elimination treatment on a care receiver in place of a person. A nursing-care robot control device (30) is a nursing-care robot control device controlling a nursing-care robot (20) performing a nursing-care behavior, including: a storage part (51) storing a behavior program; a state variable acquisition part acquiring a state variable corresponding to a state of a care receiver; and an instruction generation part generating a behavior instruction to the nursing-care robot from the behavior program and the state variable, wherein the behavior program includes an elimination treatment program for the nursing-care robot to perform an elimination treatment on the care receiver, the instruction generation part generates an elimination treatment instruction corresponding to the care receiver from the elimination treatment program as the behavior instruction, and the state variable includes a first state variable, which is a state variable used for adjusting the elimination treatment instruction, corresponding to positional data, a body type, and a posture of the care receiver.

## Description

### TECHNICAL FIELD

The present disclosure relates to a nursing-care robot system and a nursing-care robot control device.

### BACKGROUND ART

Patent Document 1 discloses a furniture apparatus combinedly used for storage/partition for making a large room serve as a semiprivate room, and a medical/care facility.

### PRIOR ART DOCUMENTS

### PATENT DOCUMENT(S)

Patent Document 1: Japanese Patent Application Laid-Open No. 2007-319186

### SUMMARY

### PROBLEM TO BE SOLVED BY THE INVENTION

An elimination treatment is one of nursing-care behaviors. The elimination treatment is a hard work and a delicate nursing care for a caregiver and a care receiver.

Accordingly, an object of the present disclosure is to be able to provide a nursing-care robot system which can perform an elimination treatment on a care receiver in place of a person.

### MEANS TO SOLVE THE PROBLEM

In order to solve the above problem, a nursing-care robot control device is a nursing-care robot control device controlling a nursing-care robot performing a nursing-care behavior, including: a storage part storing a behavior program; a state variable acquisition part acquiring a state variable corresponding to a state of a care receiver; and an instruction generation part generating a behavior instruction to the nursing-care robot from the behavior program and the state variable, wherein the behavior program includes an elimination treatment program for the nursing-care robot to perform an elimination treatment on the care receiver, the instruction generation part generates an elimination treatment instruction corresponding to the care receiver from the elimination treatment program as the behavior instruction, and the state variable includes a first state variable, which is a state variable used for adjusting the elimination treatment instruction, corresponding to positional data, a body type, and a posture of the care receiver.

### EFFECTS OF THE INVENTION

The present nursing-care robot control device controls the nursing-care robot, thus the nursing-care robot system capable of performing the elimination treatment on the care receiver in place of a person can be provided.

### BRIEF DESCRIPTION OF DRAWINGS

[Fig. 1] A schematic perspective view illustrating a nursing-care robot system according to an embodiment 1.
[Fig. 2] A functional block diagram illustrating the nursing-care robot system according to the embodiment 1.
[Fig. 3] A flow chart illustrating an elimination treatment.
[Fig. 4] A flow chart of a treatment performed by a nursing-care robot control device.
[Fig. 5] A schematic perspective view illustrating the nursing-care robot system detaching a tape.
[Fig. 6] A flow chart of a treatment performed by a nursing-care robot control device according to a third state variable.
[Fig. 7] A functional block diagram according to an imitative learning.
[Fig. 8] A functional block diagram according to an additional learning.
[Fig. 9] A functional block diagram illustrating a nursing-care robot system according to an embodiment 2.
[Fig. 10] A schematic front view illustrating a nursing-care robot according to the embodiment 2.
[Fig. 11] An explanation view illustrating the nursing-care robot according to the embodiment 2 going its rounds of a facility.
[Fig. 12] A schematic front view illustrating a modification example of the nursing-care robot according to the embodiment 2.
[Fig. 13] A functional block diagram illustrating a modification example of the nursing-care robot according to the embodiment 2.
[Fig. 14] A flow chart illustrating a facility round treatment.

### DESCRIPTION OF EMBODIMENT(S)

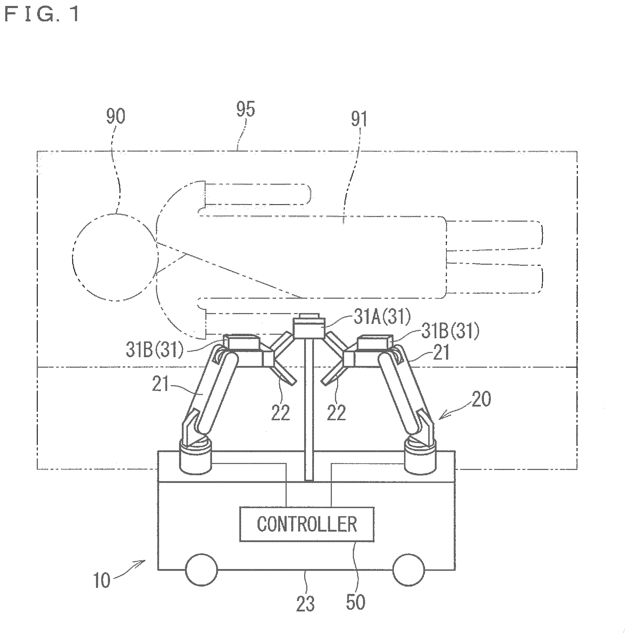

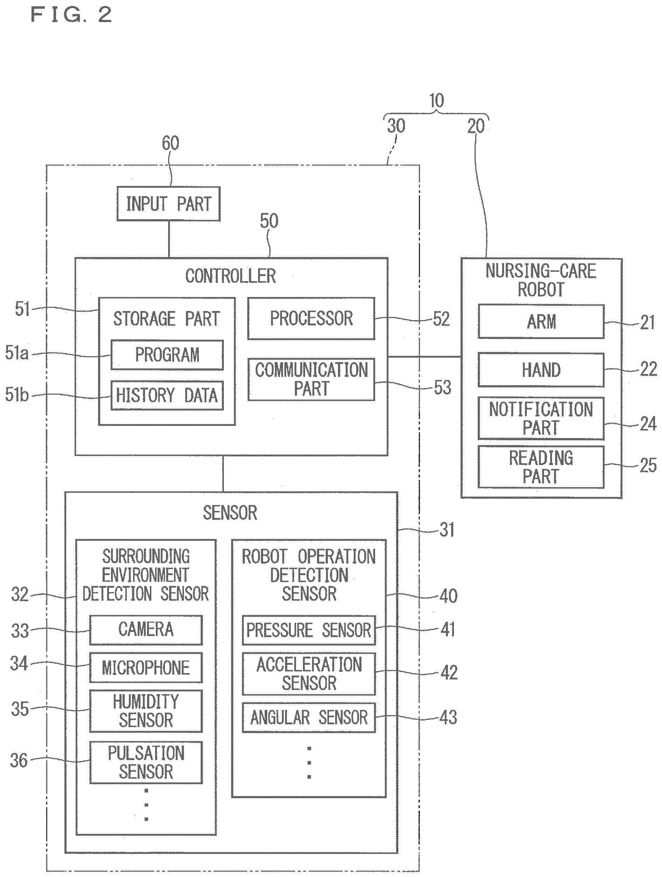

Described hereinafter is a nursing-care robot control device and a nursing-care robot system according to an embodiment. Fig. 1 is a schematic perspective view illustrating a nursing-care robot system 10 according to an embodiment 1. Fig. 2 is a functional block diagram illustrating a nursing-care robot system 10 according to the embodiment 1.

The nursing-care robot system 10 includes a nursing-care robot 20 and a nursing-care robot control device 30. The nursing-care robot 20 is controlled by the nursing-care robot control device 30 and performs a nursing-care behavior. The nursing-care robot 20 is a collaborative robot collaborating with a person.

The nursing-care robot 20 may be allocated to a hospital or a nursing-care facility, for example. In this case, a care receiver 90 is assumed to be a hospital patient in a hospital or a resident in a nursing-care facility, for example. The nursing-care robot 20 may be moved to the care receiver 90 to perform the nursing-care behavior upon request from the care receiver 90, for example. It is also applicable that the nursing-care robot 20 goes its rounds of a hospital room or a residential room, for example, to perform the nursing-care behavior on the plurality of care receivers 90 in sequence.

The nursing-care robot 20 includes at least one arm 21. The nursing-care robot 20 preferably includes the plurality of arms 21. In the example illustrated in Fig. 1, the nursing-care robot 20 includes two arms 21. A known multi-jointed robot, for example, can be adopted to the arm 21.

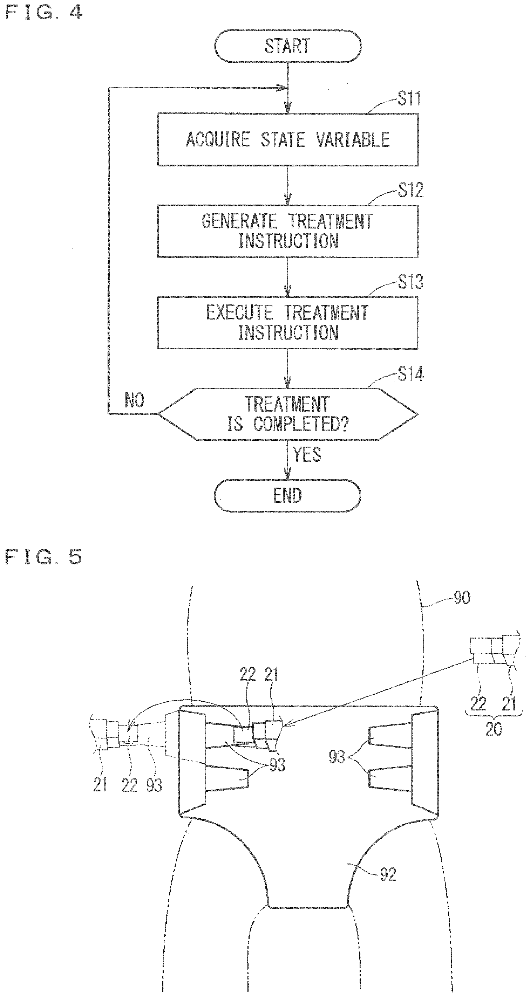

An end effector such as a hand 22 is attached to a tip end of the arm 21. The end effector is configured to be able to perform an operation required for the nursing-care behavior. Herein, at least an elimination treatment is assumed as the nursing-care behavior. In this case, the end effector is configured in accordance with an operation required for the elimination treatment. For example, it is sufficient that the end effector includes the hand 22 which can grip clothing 91 or a diaper 92 (refer to Fig. 5) to attach or detach the clothing 91 or the diaper 92 of the care receiver 90. For example, it is sufficient that the end effector includes a flexible part in which a part having contact with the care receiver 90 is flexibly formed to press or hold the care receiver 90 in changing a posture of the care receiver 90. For example, the end effector may include a device ejecting a wash solution or a device holding cloth for bed bath.

The arm 21 is attached to a base 23. When the nursing-care robot 20 is moved to the care receiver 90 from a position away from the care receiver 90 to perform the nursing-care behavior, the nursing-care robot 20 includes a movement mechanism. The movement mechanism is provided to the base 23, for example. For example, it is applicable that the movement mechanism is a driverless vehicle and the nursing-care robot 20 can autonomously travel. For example, it is applicable that the movement mechanism is a wagon and an assistant caregiver operates the wagon to move the nursing-care robot 20. When the nursing-care robot 20 performs the nursing-care behavior on the care receiver 90 moved to the nursing-care robot 20 from a position away from the nursing-care robot 20, the nursing-care robot 20 needs not include the movement mechanism.

A notification part 24 for notifying the care receiver 90 of a process to be performed may be provided to the nursing-care robot 20. The notification part 24 may be a display part displaying a character or an image or a sound transmission part for emitting sound.

A reading part 25 for reading an ID of the care receiver 90 attached to the care receiver 90 or a bed 95, for example, may be provided to the nursing-care robot 20. The ID may be a two-dimensional code or a three-dimensional code, or may also be an RFID, for example. Sex or a physical feature of the care receiver 90 may be recorded in the ID of the care receiver 90. Identification of the care receiver 90 may be recognition by various biometric authentication such as face authentication, iris authentication, fingerprint authentication, and vein authentication in addition to the ID reading, and the reading part 25 may be a reading part for these biometric authentications.

The nursing-care robot control device 30 includes at least one sensor 31 and one controller 50. Herein, the nursing-care robot control device 30 includes an input part 60 for inputting data, for example.

The sensor 31 detects a surrounding environment or an operation of the nursing-care robot 20. Herein, the sensor 31 includes a surrounding environment detection sensor 32 and a robot operation detection sensor 40.

The surrounding environment detection sensor 32 detects the surrounding environment of the nursing-care robot 20. The surrounding environment also includes the care receiver 90. The surrounding environment detection sensor 32 includes a camera 33 for acquiring image data of the surrounding environment.

The camera 33 takes an image of the surrounding environment including the care receiver 90. For example, the camera 33 includes a camera 33A provided separately from the arm 21 and a camera 33B provided to the arm 21 and moved together with the arm 21. The camera 33A is provided to the base 23 and takes an image of the surrounding environment including the care receiver 90 from a fixed point, for example. The camera 33A may panoramically take an image of the care receiver 90, for example. The camera 33A may take an image of an expression of the care receiver 90, for example. The camera 33B may take an image of an area around the hand 22, for example. It is also applicable that a stereo camera is used as the camera 33, thus the camera 33 includes a function as a distance sensor.

Moreover, the surrounding environment detection sensor 32 may include a microphone 34 for acquiring sound data, a humidity sensor 35 for acquiring humidity of the diaper 92, and a pulsation sensor 36 for acquiring pulsation of the care receiver 90, for example.

The robot operation detection sensor 40 detects the operation of the nursing-care robot 20. The robot operation detection sensor 40 includes a pressure sensor 41 detecting force received by the hand 22, an acceleration sensor 42 detecting acceleration of the arm 21, and an angular sensor 43 detecting a rotation angle of a j oint of the arm 21.

The controller 50 controls the operation of the nursing-care robot 20. The controller 50 includes a storage part 51 such as a ROM, a RAM, or an HDD, a processor 52 such as a CPU, and a communication part 53. The processor 52 reads out and executes a program 51a stored in the storage part 51, thus the controller 50 can function as various means. Assumed as the means are means of generating an instruction to the nursing-care robot 20 and means of acquiring a state variable used for the instruction. It can be considered that the controller 50 includes an instruction generation part and the state variable acquisition part as parts performing the means.

All of the controllers 50 may be or may not be provided to the nursing-care robot 20. For example, it is applicable that the communication part 53 is provided to the base 23 and the instruction generation part is provided to an upper controller connected via the communication part 53. One upper controller may control the plurality of nursing-care robots 20.

The program 51a stored in the storage part 51 includes a behavior program controlling the operation of the nursing-care robot 20. The behavior program includes an elimination treatment program for the nursing-care robot 20 to perform the elimination treatment on the care receiver 90. The behavior program may be or may not be a mechanically learned program. When the behavior program is a mechanically learned program, any method is applicable as the mechanical learning. The behavior program may be or may not be updated by an additional learning based on an operation in an actual nursing-care behavior of the nursing-care robot 20.

The state variable acquisition part acquires a state variable corresponding to a state of the care receiver 90. The state variable is used for adjusting the behavior instruction. Herein, the state variable includes a first state variable, a second state variable, and a third state variable. The first state variable, the second state variable, and the third state variable are used for adjusting the elimination treatment instruction. The state variable may be an output itself of a sensor, or may also be acquired by analyzing an output of a sensor. The state variable acquisition part acquires the output of the sensor or acquires and analyzes the output of the sensor.

The first state variable is a state variable corresponding to positional data, a body type, or a posture of the care receiver 90. The positional data is positional data of a specific part of the care receiver 90 with respect to each of the nursing-care robot 20 and the bed 95, for example. The specific part is a face, a hand, or a toe, for example. The positional data may be acquired by performing image analysis on an acquired image taken by the camera 33. In this case, the state variable acquisition part includes an image analysis part for determining the positional data from the image taken by the camera 33. The body type and the posture may be determined from the image taken by the camera 33 or the positional data. The body type is a variable having a smaller change than the positional data and the posture. The body type may be acquired by input from the input part 60 or reading of the ID. In a case of a second or subsequent elimination treatment on the specific care receiver 90, the body type may be acquired from past history data 51b.

The second state variable is a state variable corresponding to sex of the care receiver 90. The sex may be acquired by performing image analysis on an acquired image taken by the camera 33. The sex may be acquired by input from the input part 60 or reading of the ID. In a case of a second or subsequent elimination treatment on the specific care receiver 90, the sex may be acquired from the past history data 51b.

The third state variable is a state variable corresponding to feeling of the care receiver 90 in the elimination treatment behavior of the nursing-care robot 20. The feeling of the care receiver 90 is analyzed and acquired from an index value of an expression of the care receiver 90, sound emitted from the care receiver 90, and change of pulsation of the care receiver 90, for example. In this case, the state variable acquisition part includes a feeling analysis part for determining feeling data from the index value of the feeling. The index value of the expression, the emitted sound, and the change of pulsation of the care receiver 90 is detected by a feeling index value detection sensor. The feeling index value detection sensor includes the camera 33 taking an image of an expression of the care receiver 90, the microphone 34 taking in sound emitted from the care receiver 90, and the pulsation sensor 36 measuring pulsation of the care receiver 90, for example. The feeling analysis part is made up of a known feeling analysis program using an index value detected by the feeling index value detection sensor.

The feeling may be acquired from one index value or a plurality of index values. When the feeling is acquired from the plurality of index values, a weight of each of the plurality of index values may be equivalent to each other, or different weights may be assigned to the plurality of index values, respectively.

The instruction generation part generates the behavior instruction to the nursing-care robot 20 from the behavior program. The instruction generation part generates the elimination treatment instruction corresponding to the care receiver 90 as the behavior instruction based on the elimination treatment program and the state variable acquired by the state variable acquisition part.

### <Flow of elimination treatment>

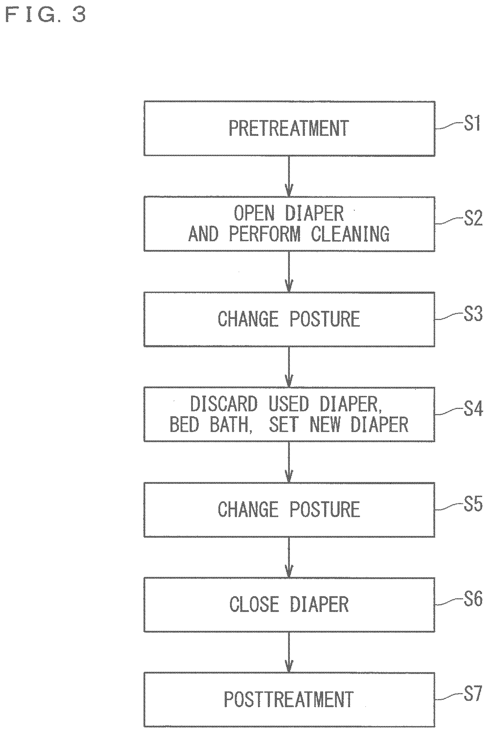

A flow of the elimination treatment is described. Fig. 3 is a flow chart illustrating the elimination treatment. Assumed in the elimination treatment illustrated in Fig. 3 is a case where a person changes the diaper 92 with a tape 93 for the bedridden care receiver 90 wearing the diaper 92. For example, it is applicable that the nursing-care robot 20 is made to perform the elimination treatment illustrated in Fig. 3 as it is. The elimination treatment illustrated in Fig. 3 may be partially improved so as to be appropriate as the elimination treatment performed by the nursing-care robot 20.

Firstly, a pretreatment is performed in Step S1. For example, assumed as the pretreatment are a treatment of setting the care receiver 90 to a state appropriate for the elimination treatment, a treatment of determining whether or not the diaper 92 needs to be changed, and a treatment of setting the new diaper 92 to a state of being easily set.

The state of the care receiver 90 appropriate for the elimination treatment is a state where a comforter on the care receiver 90 is removed, a body position of the care receiver 90 is a supine posture, hands of the care receiver 90 is located on a chest, and the clothing 91 of the care receiver 90 is displaced or detached so that the diaper 92 of the care receiver 90 is exposed. It is determined whether the state of the care receiver 90 is the state appropriate for the elimination treatment, and when the care receiver 90 is not in the state appropriate for the elimination treatment, the care receiver 90 is set to the state appropriate for the elimination treatment.

When the diaper 92 is the diaper 92 in which a color is changed in accordance with presence or absence of the elimination, for example, necessity of change of the diaper 92 is determined by detecting change of the color. The necessity thereof may be determined by having contact with the diaper 92 and detecting a degree of wetness. The determination whether the diaper 92 needs to be changed may be omitted in a case where the diaper 92 is changed upon request from the care receiver 90 to change the diaper 92, for example.

The treatment of setting the new diaper 92 to the state of being easily set is a treatment of unfolding the folded new diaper 92 so that that the diaper 92 is easily set or attaching a urine absorption pad to the new diaper 92 as necessary.

In next Step S2, the tape 93 of the diaper 92 which the care receiver 90 wears is detached and a front part of the diaper 92 is opened. Then, a genital area is cleaned. At this time, it is confirmed whether there is stool, and a subsequent treatment is changed in accordance with presence or absence of the stool.

In next Step S3, the posture of the care receiver 90 is changed from a supine posture to a decubitus posture. At this time, a specific part of the care receiver 90 is pressed or held to change the posture of the care receiver 90 from the supine posture to the decubitus posture.

In next Step S4, the used diaper 92 is removed from the bed 95 and discarded. Bed bath is performed on the care receiver 90. The new diaper 92 is put on the bed 95 and set to a predetermined position with respect to the care receiver 90. The predetermined position with respect to the care receiver 90 is a position where a center of the diaper 92 in a width direction coincides with a center of the care receiver 90 when the posture of the care receiver 90 is returned to the supine posture. It is also a position where an upper end of the diaper 92 is located around a lower abdomen of the care receiver 90.

In next Step S5, the posture of the care receiver 90 is changed from the decubitus posture to the supine posture. At this time, a specific part of the care receiver 90 is pressed or held to change the posture of the care receiver 90 from the decubitus posture to the supine posture.

In next step S6, the new diaper 92 is closed and positioned with respect to the care receiver 90. At this time, the diaper 92 is disposed along an inguinal part to prevent a gap. A shape of a gathering part of the diaper 92 is arranged. Then, the tape 93 of the diaper 92 is applied.

In next Step S7, a posttreatment is performed. For example, a treatment of arranging a shape of the clothing 91 of the care receiver 90 and a treatment of applying a comforter are assumed as the posttreatment.

Step S1 may be partially or wholly performed by a person or the nursing-care robot 20. Step S7 may be partially or wholly performed by a person or the nursing-care robot 20. It is preferable that the nursing-care robot 20 performs at least the processes associated with exposure of a genital area from opening of the diaper 92 in Step S2 to closing of the diaper 92 in Step S6. The nursing-care robot 20 may perform all of Step S1 to Step S7.

### <Flow chart of treatment performed by nursing-care robot control device 30>

Fig. 4 is a flow chart of a treatment performed by the nursing-care robot control device 30. The nursing-care robot control device 30 classifies each operation in each step of Step S1 to Step S7 into a plurality of small processes, and performs the treatment in Fig. 4 for each of the plurality of small processes, for example. Specifically, in Step S2, for example, each operation is classified into a process of making the hand 22 detaching the tape 93 in the operation of opening the diaper 92, a process of making the hand 22 grip and move the front part of the diaper 92, and a process of cleaning the genital area. Then, it is determined as illustrated in Fig. 4 whether a necessary state variable is acquired, the treatment instruction is generated, executed, and completed in each process.

This treatment is described more specifically using the process of making the hand 22 detach the tape 93 as an example. Fig. 5 is a diagram illustrating the nursing-care robot system 10 detaching the tape 93.

Firstly, as Step S 11, a position and a direction of the tape 93 of the diaper 92 are acquired as a state variable in the first state variable described above. The position and the direction of the tape 93 of the diaper 92 may be acquired based on the image of the camera 33.

The treatment instruction is generated as next Step S12. Herein, the treatment instruction includes a position and a direction of the hand 22 which has been moved to a movement destination to grip the tape 93, a position of the hand 22 which has been moved to a movement destination while gripping the tape 93, and a timing of an open-close motor of the hand 22, for example. The position and the direction of the hand 22 which has been moved to the movement destination to grip the tape 93 are acquired in accordance with the position and the direction of the tape 93 of the diaper 92 acquired in Step S11. The position of the hand 22 which has been moved to the movement destination while gripping the tape 93 is a lateral position of the care receiver 90 close to a back side, for example, and is acquired from the first state variable described above, for example. At this time, it is determined based on the first state variable whether the nursing-care robot 20 has contact with the care receiver 90 when the hand 22 is moved from a current position to the position of the movement destination. When the nursing-care robot 20 is predicted to have contact with the care receiver 90, a route is adjusted to suppress the contact of the nursing-care robot 20 with the care receiver 90.

As next Step S13, the treatment instruction generated in Step S12 is outputted to the nursing-care robot 20, and is executed.

As next Step S14, it is determined whether the tape 93 is detached. The determination may be performed based on the image taken by the camera 33, for example. When the tape 93 is detached, it is determined that the process of making the hand 22 detach the tape 93 is completed, and the process proceed to the next process. When the tape 93 is not detached, the process is returned to Step S11, and the process of making the hand 22 detach the tape 93 is performed again.

Also regarding the other process, the processes corresponding to Step S1 1 to Step S14 are repeated, thus the nursing-care robot control device 30 controls the nursing-care robot 20 and the nursing-care robot 20 can be made to change the diaper 92 of the care receiver 90.

The first state variable described above may be used for adjusting force of pressing the care receiver 90 in changing the posture and adjusting a position of setting the diaper 92, for example, in addition to the adjustment of the movement position and the movement route of the hand 22. The second state variable described above may be used for adjusting a position of attaching a urine absorption pad and a method of cleaning and performing bed bath on a genital area, for example.

### <Flow chart of treatment performed by nursing-care robot control device according to third state variable>

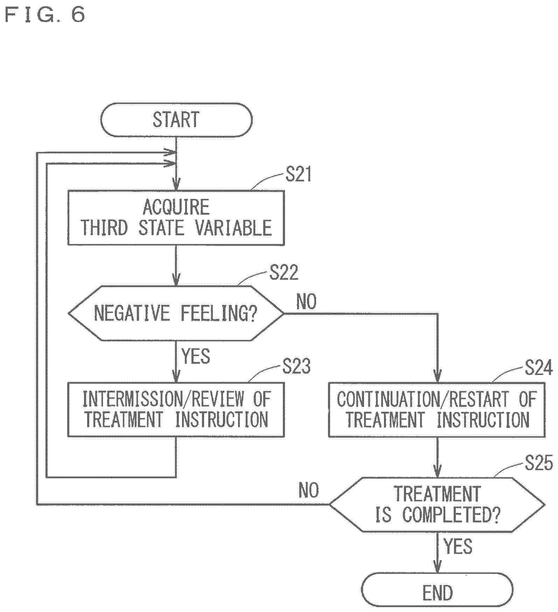

Fig. 6 is a flow chart of a treatment performed by the nursing-care robot control device 30 according to a third state variable.

Firstly, in Step S21, feeling of the care receiver 90 as the third state variable is acquired.

In next Step S22, it is determined whether the feeling of the care receiver 90 is negative feeling. When it is determined to be the negative feeling, the process proceeds to Step S23, and when it is not determined to be the negative feeling, the process proceeds to Step S24.

When it is determined to be the negative feeling and the process proceeds to Step S23, at least one of intermission and/or review of the treatment instruction is performed in Step S23. When the intermission is performed, driving of the nursing-care robot 20 is stopped for a predetermined period of time, for example. When the review is performed, the treatment instruction is adjusted to reduce a speed and acceleration relating to the operation of the nursing-care robot 20. When Step S23 is completed, the process is returned to Step S21.

When it is not determined to be the negative feeling and the process proceeds to Step S24, the treatment instruction is continued, and the process proceeds to Step S25.

In next Step S25, it is determined whether the process is completed, and when the process is completed, the process proceeds to the next process. When the process is not completed, the process is returned to Step S21.

In this manner, the acquisition of the feeling of the care receiver 90 and the determination whether the feeling is the negative feeling are repetitively performed until the process is completed, thus nursing care considerate to the feeling of the care receiver 90 can be provided.

### <Imitative learning>

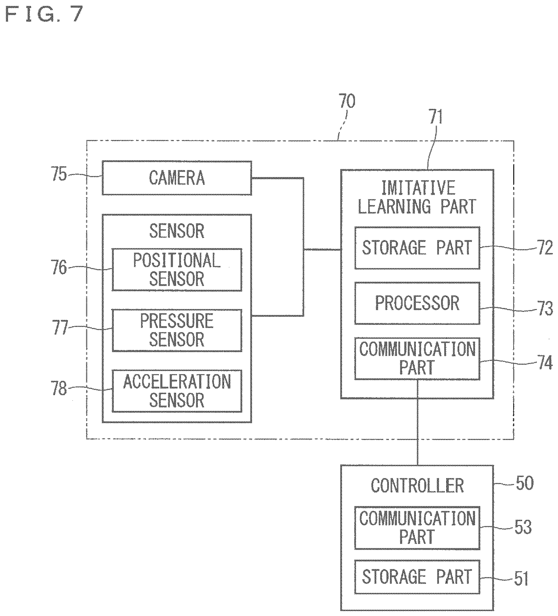

The elimination treatment program may be a program mechanically learned previously to imitate a model nursing-care behavior based on model nursing-care data in the model nursing-care behavior. Fig. 7 is a functional block diagram according to an imitative learning.

A person as the caregiver may directly perform the model nursing-care behavior on a model care receiver. A person may operate the nursing-care robot 20 to perform the model nursing-care behavior on the model care receiver. The model nursing-care behavior may be performed on the model care receivers having the first state variables described above different from each other. An imitative learning device 70 performs the imitative learning of the model nursing-care behavior. The imitative learning device 70 includes an imitative learning part 71, a camera 75, and sensors 76, 77, and 78 provided to a nursing-care hand.

The imitative learning part 71 includes a storage part 72 such as a ROM, a RAM, or an HDD, a processor 73 such as a CPU, and a communication part 74, for example. The processor 73 reads out and executes a program stored in the storage part 72, thus the imitative learning part 71 can function as various means. Herein, the imitative learning part 71 is provided separately from the controller 50, and can have communication with the controller 50 via the communication part 74. The imitative learning part 71 transmits the elimination treatment program to the controller 50 via the communication part 74. The imitative learning part 71 may be provided to the controller 50.

The sensors 76, 77, and 78 includes a positional sensor 76, a pressure sensor 77, and an acceleration sensor 78, for example. When a person as the caregiver directly performs the model nursing-care behavior on the model care receiver, the nursing-care hand is a hand of the caregiver. In this case, each of the sensors 76, 77, and 78 is attached to the caregiver. When a person operates the nursing-care robot 20 and directly performs the model nursing-care behavior on the model care receiver, the nursing-care hand is the hand 22 of the nursing-care robot 20. In this case, each of the sensors 76, 77, and 78 is attached to the nursing-care robot 20.

The model nursing-care data includes outline data of the model care receiver in the model nursing-care behavior. The outline data is acquired by analyzing an image of the model care receiver taken by the camera 75, for example.

The model nursing-care data includes coordinate data of the nursing-care hand in the model nursing-care behavior. The coordinate data is data of a relative coordinate relative to the model care receiver. The coordinate data is acquired from an output from the positional sensor 76 and the outline data of the model care receiver. The model nursing-care data includes at least one piece of data in the acceleration sensor 78 and/or the pressure sensor 77.

The imitative learning part 71 imitates the model nursing-care behavior based on the model nursing-care data in each process. The processor 73 calculates a parameter for imitating the model nursing-care behavior based on the model nursing-care data. Specifically, in the process of detaching the tape 93 described above, acquired are the position and the direction of the hand 22 in gripping the tape 93, the position of the movement destination of the hand 22 while gripping the tape 93, and the route thereto based on the outline data and the coordinate data, for example. A movement speed of the hand 22 is acquired based on data of the acceleration sensor 78. Grip force or press force of the hand 22 is acquired based on data of the pressure sensor 77.

Herein, the mechanical learning is normally roughly divided into three types of learning of supervised learning, unsupervised learning, and reinforcement learning. Also applicable is mechanical learning using neural network such as deep learning or mechanical learning in which neural network such as deep learning is not used. Applicable as an algorithm of the mechanical learning is linear regression, logistic regression, decision tree, random forest, K-nearest neighbor algorithm (KNN), K-means clustering (K-means), support vector machine (SVM), support vector regression (SVR), naive bayes, CNN, RNN, or GAN, for example. The mechanical learning imitating the model nursing-care behavior may be any of supervised learning, unsupervised learning, and enforce learning. Different learning in supervised learning, unsupervised learning, and enforce learning may be performed in each process. A method of imitative learning is not particularly limited, however, behavior cloning, expert data fusion, or inverse reinforcement learning is also applicable.

### <Additional learning>

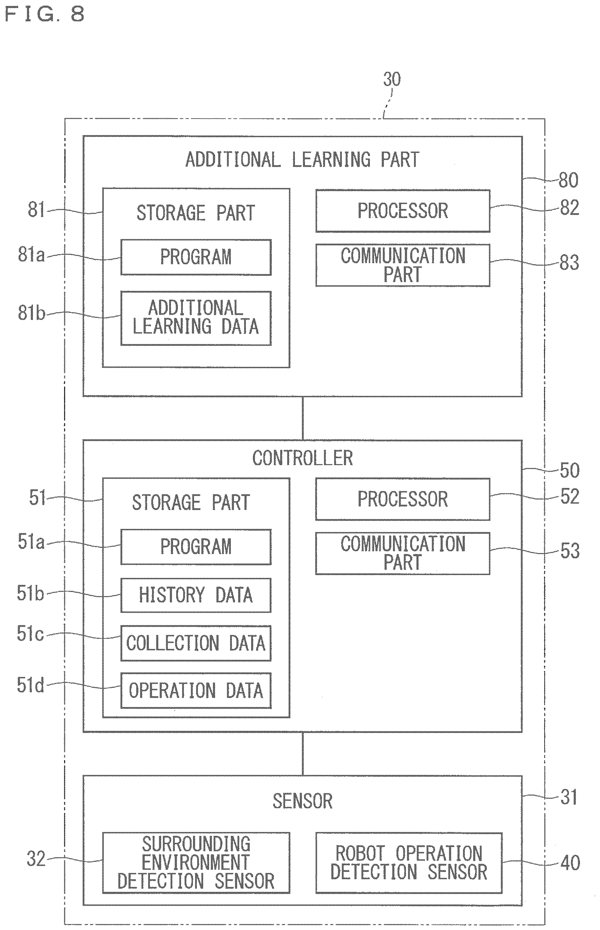

The elimination treatment program may be additionally learned based on the elimination treatment on the care receiver 90 by the nursing-care robot 20 and updated. Fig. 8 is a functional block diagram illustrating the nursing-care robot control device 30 in a case where additional learning is performed.

The nursing-care robot control device 30 includes an additional learning part 80. The additional learning part 80 outputs an elimination treatment program updated based on a state variable acquired by the state variable acquisition part in the elimination treatment behavior of the nursing-care robot 20 on the care receiver 90. The additional learning part 80 includes a storage part 81 such as a ROM, a RAM, or an HDD, a processor 82 such as a CPU, and a communication part 83, for example. The processor 82 reads out and executes a program 81a stored in the storage part 81, thus the additional learning part 80 can function as various means. Herein, the additional learning part 80 is provided separately from the controller 50, and can have communication with the controller 50 via the communication part 83. The additional learning part 80 may be provided to the controller 50.

The controller 50 transmits additional learning data 81b in which collection data 51c in the elimination treatment on the care receiver 90 by the nursing-care robot 20 and operation data 51d are associated with each other to the additional learning part 80. The collection data 51c is a state variable directly acquired from an output of the sensor 31 or a state variable acquired by analyzing the output of the sensor 31. The operation data 5 1d is operation data of the nursing-care robot 20. The additional learning part 80 performs additional learning for at least one process based on the additional learning data 81b, and updates the elimination treatment program. The updated elimination treatment program is transmitted to the controller 50 via the communication part 83. The additional learning is reinforcement learning. An algorithm of the additional learning is not particularly limited, however, the algorithm described above, for example, can be used.

At this time, it is also applicable that the additional learning part 80 performs reinforcement learning in reward for the third state variable, that is to say, feeling of the care receiver 90 as the additional learning, and updates the elimination treatment program. In this case, the additional learning part 80 may optimize the elimination treatment program so that negative feeling of the care receiver 90 is reduced and positive feeling is increased.

### <Effect etc.>

According to the nursing-care robot control device 30 having such a configuration, the elimination treatment instruction corresponding to the care receiver 90 is generated from the elimination treatment program using the first state variable corresponding to the positional data, the body type, and the posture of the care receiver 90, thus the nursing-care robot 20 can be made to perform the elimination treatment in accordance with the body type and the posture of the care receiver 90. Accordingly, the nursing-care robot 20 can perform the elimination treatment which is a hard and delicate nursing-care behavior in place of a person.

The elimination treatment instruction corresponding to the care receiver 90 is generated from the elimination treatment program using the second state variable corresponding to sex data of the care receiver 90 in addition to the first state variable. Accordingly, the nursing-care robot system 10 can provide more appropriate nursing care in accordance with the sex of the care receiver 90.

The elimination treatment instruction corresponding to the care receiver 90 is generated from the elimination treatment program using the third state variable corresponding to feeling of the care receiver 90 in the elimination treatment behavior of the nursing-care robot 20 in addition to the first state variable. Accordingly, the nursing-care robot system 10 can provide more appropriate nursing care in accordance with the feeling of the care receiver 90.

It is also applicable that the elimination treatment program is a program mechanically learned to imitate the model nursing-care behavior based on the model nursing-care data in the model nursing-care behavior, and the model nursing-care data includes the coordinate data of the nursing-care hand and the outline data of the model care receiver in the model nursing-care behavior. In this case, accuracy of the elimination treatment program is improved.

When the data of at least one of the acceleration sensor 78 and/or the pressure sensor 77 attached to the nursing-care hand is used as the nursing-care data, accuracy of degree of force in the nursing-care robot 20 is thereby improved.

When the nursing-care robot control device 30 further includes the additional learning part 80 outputting the elimination treatment program updated based on the state variable acquired by the state variable acquisition part in the elimination treatment behavior of the nursing-care robot 20 on the care receiver 90, accuracy of the elimination treatment program is improved more as the elimination treatment behavior is repetitively performed by the nursing-care robot 20.

### [Embodiment 2]

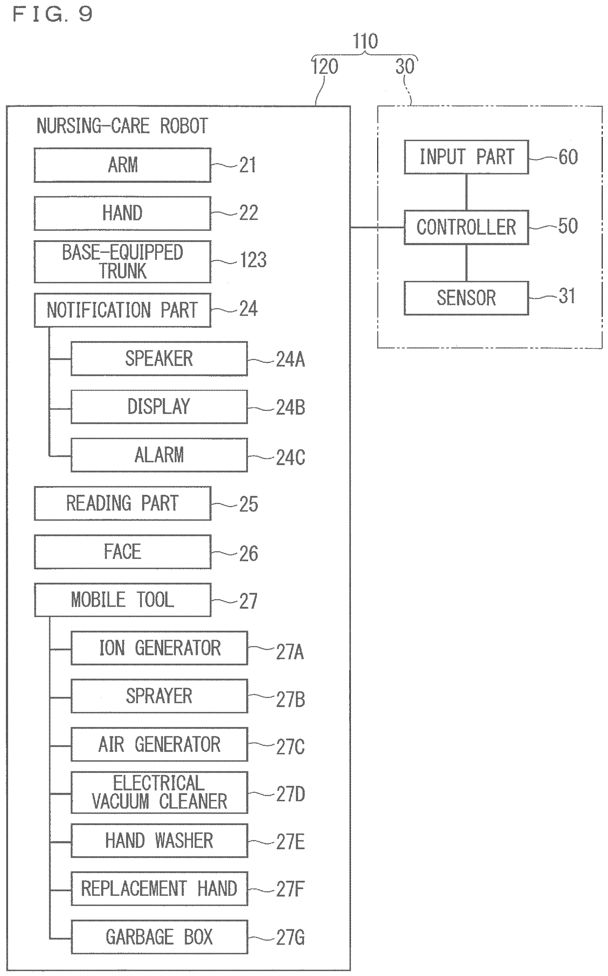

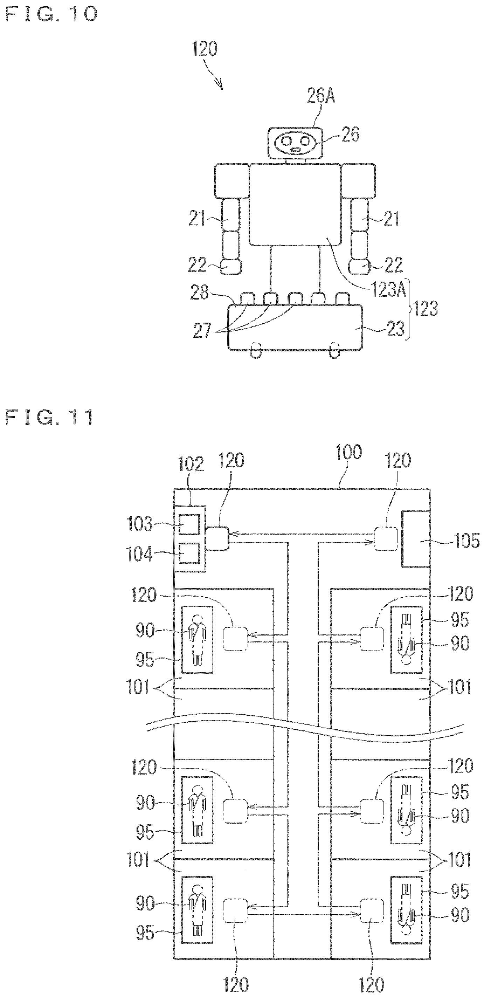

Described is a nursing-care robot system according to an embodiment 2. Fig. 9 is a functional block diagram illustrating a nursing-care robot system 110 according to the embodiment 2. Fig. 10 is a schematic front view illustrating a nursing-care robot 120 according to the embodiment 2. Fig. 11 is an explanation view illustrating the nursing-care robot 120 according to the embodiment 2 going its rounds of a facility 100. In the description of the present embodiment, the same signs are assigned to constituent elements similar to those described above, and the description thereof is omitted.

In the nursing-care robot system 110 according to the present embodiment, the nursing-care robot 120 is formed into a human-like shape. In the present disclosure, the human-like nursing-care robot 120 needs not completely coincide with an appearance of a person, however, it is sufficient that the nursing-care robot 120 has an appearance which can be recognized to have the human-like shape when a person such as the care receiver 90 sees the nursing-care robot 120. The nursing-care robot 120 includes a trunk 123A, the arm 21 extending from the trunk 123A, the hand 22 provided to the arm 21, and a face 26.

The trunk 123A is a part above a waist, for example. The trunk 123A is integrally formed with the base 23 which can travel by itself to constitute a base-equipped trunk 123. In the example illustrated in Fig. 10, the base 23 is wagon with wheels. The base 23 rotates the wheels, thereby traveling. A drive part such as an electrical motor and a transmission mechanism such as a gear are provided to the base 23 to rotate the wheels. The nursing-care robot control device 30 controls the drive part to make the nursing-care robot travel by itself. The nursing-care robot 120 may include a battery supplying electrical power to the electrical motor, and the battery may be provided to the base. The base 23 may not be the wagon. The base 23 may be legs with which the nursing-robot 120 can walk.

The arm 21 and the hand 22 need not also completely coincide with a person, but are preferably formed into a shape representing a person as much as possible. It is sufficient that the pair of arms 21 are provided in direction opposite to each other from an upper part of the trunk 123A toward a lateral side. It is sufficient that the hand 22 is provided to a tip end of the arm 21.

A position and a configuration of the face 26 are optionally set in the nursing-care robot 120. It is sufficient that the face 26 can be recognized to represent a face of a person when a person such as the care receiver 90 sees the face 26. In the example illustrated in Fig. 10, the nursing-care robot 120 includes a head 26A, and the face 26 is provided to a surface of the head 26A. It is also applicable that a human-like robot does not include the head 26A but the face 26 is provided to a surface of the trunk 123A. It is optionally set which parts the face 26 includes, eyes, a mouth, and a mouth, for example, which are parts included in a normal person. The face 26 preferably includes eyes and a mouth. It is sufficient that the parts of the face 26 has an appearance representing the parts of a person, thus need not have functions which a normal person has. For example, it is sufficient that the eyes in the face 26 has an appearance representing eyes of a person, thus may or may not include an image recognition means. For example, it is sufficient that the mouth in the face 26 has an appearance representing a mouth of a person, thus may or may not include a sound generation means. The parts of the face 26 may be directly formed as a part of a body of the nursing-care robot 120. It is also applicable that the nursing-care robot 120 includes an image display part such as a display 24B in a part serving as the face 26, and the parts of the face are displayed on the image display part.

It is also applicable that the nursing-care robot 120 can change the appearance of the face 26 so that a person seeing the face 26 can recognize change of an expression, and the nursing-care robot control device 30 performs control so that the appearance of the face 26 is changed. For example, when the parts of the face 26 are directly formed as a part of the body of the nursing-care robot 120, it is applicable that the nursing-care robot 120 can move the parts to express change of the expression, and the nursing-care robot control device 30 performs control so that the parts are appropriately moved. For example, when the parts of the face 26 are displayed on the image display part such as the display 24B, it is also applicable that the nursing-care robot 120 can change an image of the parts of the face 26 to express change of the expression, and the nursing-care robot control device 30 performs control so that the image of the parts is appropriately changed.

As illustrated in Fig. 11, the nursing-care robot 120 is allocated in the facility 100 for admitting the plurality of care receivers 90 such as a nursing-care facility. In the example illustrated in Fig. 11, the facility 100 includes a plurality of residential rooms 101 for individually admitting the plurality of care receivers 90, respectively. The residential room 101 in the facility 100 may be configured to admit the plurality of care receivers 90 in one room.

The nursing-care robot 120 goes its rounds of the plurality of care receivers 90 in sequence in the facility 100. The round may be set to be performed at a fixed time in a day. The round may be performed once or several times in a day. The nursing-care robot 120 changes the diaper 92 when the nursing-care robot 120 visits to each of the plurality of care receivers 90. The nursing-care robot 120 visits to the care receiver 90 in each residential room 101 in sequence, and changes the diaper 92 of the care receiver 90 on the bed 95 in each residential room 101.

A robot station 102 serving as a waiting position of the nursing-care robot 120 may be provided to the facility 100. A power supply part 103 supplying electrical power to the nursing-care robot 120 may be provided to the robot station 102. The power supply part 103 may be a charging station for charging the nursing-care robot 120 or a rechargeable battery pack for changing a rechargeable battery pack of the nursing-care robot 120, for example.

Supply management station 104 managing supplies used by the nursing-care robot 120 in the nursing-care operation may be provided to the facility 100. The supplies include disposable consumable supplies such as the diaper 92, an air refresher, and an antiseptic agent. The supplies may include a linen replacement such as a sheet and a towel. The nursing-care robot 120 may restock the supplies by itself from the supply management station 104. A person may restock the nursing-care robot 120 with the supplies. When there is a decreasing stock of the supplies in the supply management station 104, a person such as a manager of the facility 100 may restock the supply management station 104 with the supplies. When the nursing-care robot 120 manages an amount of stock in the supply management station 104 and there is a decreasing stock, the nursing-care robot 120 may notify the manager of the facility 100 of shortage of stock. The manager may be a manager in the facility 100, a manager outside the facility 100, or both the manager in the facility 100 and the manager outside the facility 100. The same applies to the manager described hereinafter.

A garbage pickup station 105 where garbage is disposed may be provided to the facility 100. The nursing-care robot 120 carries a garbage box 27G in the round and collects garbage in the garbage box 27G. The nursing-care robot 120 transfers the garbage in the mobile garbage box 27G to the garbage pickup station 105 at an appropriate time such as a time of finishing the round. The garbage pickup station 105 may be dedicated to the nursing-care robot 120, may also be shared with a person. The garbage in the garbage pickup station may be picked up by a manager or a garbage collector, for example.

The nursing-care robot control device 30 controls the nursing-care robot 120 so that the nursing-care robot 120 has communication with the care receiver 90. The nursing-care robot system 110 includes a text acquisition part acquiring a text transmitted from the care receiver 90, a text output part outputting a text to the care receiver 90, and a text creation part creating a text outputted from the text output part. When the text creation part creates the text, in a case there is a text acquired by the text acquisition part, the text creation part creates the text outputted from the text output part based on the text acquired by the text acquisition part.

The acquisition method in the text acquisition part and the output method in the text output part in the nursing-care robot 120 are not particularly limited, however, sound, a written message, or sign language is also applicable. The camera 33 or the microphone 34 described above, for example, can be the text acquisition part. The notification part 24 described above can be the text output part. A method of outputting the text (method of transmitting communication) may be different between the nursing-care robot 120 and the care receiver 90. It is sufficient that the nursing-care robot 120 includes a plurality of text output parts corresponding to a plurality of transmission methods. It is sufficient that the nursing-care robot 120 includes a plurality of text acquisition parts corresponding to a plurality of receiving methods. When the text is acquired or outputted via sound or an image, it is sufficient that the nursing-care robot system 110 includes a text conversion part converting the text and the sound or the image.

Specifically, the text acquisition part by the sound in the nursing-care robot 120 may include a sound acquisition part such as the microphone 34, for example. The text acquisition part by the written message in the nursing-care robot 120 may include an image recognition means such as the camera 33 or a text input means such as a keyboard or a touch pad through which the care receiver 90 can directly input the text. The text acquisition part by the sign language in the nursing-care robot 120 may include an image recognition means such as the camera 33.

The text output part by the sound in the nursing-care robot 120 may include a sound generation part such as a speaker 24A, for example. The text output part by the written message in the nursing-care robot 120 may include an image display part such as the display 24B displaying the text, or may also include the hand 22 actually writing the text on paper, for example. The text output part by the sign language in the nursing-care robot 120 may include the hand 22 actually expressing the sign language, or may also include an image display part such as the display 24B displaying computer graphics (CG) expressing the sign language, for example.

The nursing-care robot 120 and the care receiver 90 may have communication with each other via a terminal owned by the care receiver 90. In this case, the nursing-care robot 120 may include a wired or wireless communication means to have communication with the terminal of the care receiver 90. For example, the text output part may transmit the text to the terminal owned by the care receiver 90. The text acquisition part may receive the text inputted by the care receiver 90 to the terminal owned by the care receiver 90.

The nursing-care robot 120 may appropriately select the method from the plurality of methods of outputting the text and the plurality of method of acquiring the text and use the method in accordance with the care receiver 90. It is also appliable that the nursing-care robot 120 acquires output method information of the text and acquisition method information of the text corresponding to the care receiver 90 and transmits and receive the text to and from the care receiver 90 by the output method and acquisition method based on the output method information and the acquisition method information. For example, the nursing-care robot 120 may acquire transmission method information and receiving method information from a reply from the care receiver 90 at a time of asking a question of a favorite output method and a favorite acquisition method of the care receiver 90. It is also applicable that the output method information and the acquisition method information which are desired or can be taken by the care receiver 90 are stored in an ID of the care receiver 90, and the nursing-care robot 120 reads the ID of the care receiver 90, thereby acquiring the output method information and the acquisition method information which is desired or can be taken by the care receiver 90.

The text creation part in the nursing-care robot system 110 may be provided to the nursing-care robot 120, or may also be provided separately from the nursing-care robot 120 so as to be able to have communication with the nursing-care robot 120. The text creation part may create the text using a chat artificial intelligence (AI) or a chatbot, for example. The text creation part may be able to have communication with the chat Ai or the chatbot, for example, provided separately from the nursing-care robot system 110. The nursing-care robot system 110 itself may include the chat Ai or the chatbot, for example.

In a case where the nursing-care robot 120 detects abnormality of the nursing-care robot 120 itself, the nursing-care robot control device 30 controls the nursing-care robot 120 so that the nursing-care robot 120 transmits notification to a manager. Such a configuration functions as a security function or a mischief prevention function for the nursing-care robot 120 in the nursing-care robot system 110. Herein, the case where the nursing-care robot 120 detects the abnormality of the nursing-care robot 120 itself indicates a case where the nursing-care robot 120 cannot exclude the abnormality by itself and cannot perform a normal treatment. Assumed as the case where the nursing-care robot 120 cannot exclude the abnormality by itself and cannot perform the normal treatment is a case where a lack of supplies necessary for the nursing-care robot 120 to change the diaper 92 causes obstacle to the change of the diaper 92 or a case where the nursing-care robot 120 cannot be moved by reason that the nursing-care robot 120 falls or there is an obstruction on a passage, for example. The nursing-care robot 120 may notify a manager of abnormality using an alarm 24C in the notification part 24, for example. The nursing-care robot control device 30 can appropriately control the behavior of the nursing-care robot 120 after notifying the manager of the abnormality. For example, the nursing-care robot control device 30 may make the nursing-care robot 120 stand still until the manager corrects the abnormality. For example, the nursing-care robot control device 30 may make the nursing-care robot 120 perform behavior other than the behavior relating to the abnormality. At this time, different control may be performed depending on a degree of abnormality, or uniform control may also be performed regardless of the degree of abnormality.

The nursing-care robot control device 30 controls the nursing-care robot 120 so that the nursing-care robot 120 transmits notification to a manager when the nursing-care robot 120 detects abnormality of a surrounding environment. Accordingly, the nursing-care robot 120 functions as a security function for the facility 100 in the nursing-care robot system 110, for example. Herein assumed as the case where the nursing-care robot 120 detects the abnormality of the surrounding environment is abnormality occurring in the care receiver 90 or abnormality occurring in a person in the facility 100 or equipment, for example. For example, vomiting or a newly-detected disease is assumed as the abnormality occurring in the care receiver 90. Assumed as the abnormality occurring in the person in the facility 100 is a case where a person crouches down to a passage or a person asks for help from the nursing-care robot 120, for example. A trouble or breakdown of the equipment is assumed as the abnormality occurring in the equipment in the facility 100, for example. The nursing-care robot 120 may notify a manager of abnormality using the alarm 24C in the notification part 24, for example. The nursing-care robot control device 30 can appropriately control the behavior of the nursing-care robot 120 after notifying the manager of the abnormality. For example, the nursing-care robot control device 30 may make the nursing-care robot 120 stand still until the manager corrects the abnormality. For example, the nursing-care robot control device 30 may make the nursing-care robot 120 perform behavior other than the behavior relating to the abnormality. At this time, different control may be performed depending on a degree of abnormality, or uniform control may also be performed regardless of the degree of abnormality.

The nursing-care robot 120 includes a mobile tool 27. The mobile tool 27 is an attached tool carried by the nursing-care robot 120 when the nursing-care robot 120 visits to the care receiver 90 to change the diaper 92. In the example illustrated in Fig. 9, the nursing-care robot 120 includes, as the mobile tool 27, an ion generator 27A, a sprayer 27B, an air generator 27C, an electrical vacuum cleaner 27D, a hand washer 27E, a replacement hand 27F, and the garbage box 27G. The nursing-care robot 120 may include, as the mobile tool 27, only some of the ion generator 27A, the sprayer 27B, the air generator 27C, the electrical vacuum cleaner 27D, the hand washer 27E, the replacement hand 27F, and the garbage box 27G. For example, it is applicable that some of the tools such as the hand washer 27E and the replacement hand 27F are not carried but are disposed in a fixed position such as the robot station 102, and are used in the fixed position such as the robot station 102. The mobile tool 27 may include the mobile tool 27 incorporated as a part of the nursing-care robot 120. The mobile tool 27 may include the mobile tool 27 which is a manufacture different from the nursing-care robot 120 and is simply carried and used by the nursing-care robot 120.

The ion generator 27A, the air generator 27C, and the electrical vacuum cleaner 27D, for example, in the mobile tool 27 are electrical tools. The electrical tool may be a battery drive type tool or a type of tool directly connected to a commercial power source and driven. When the electrical tool is the battery drive type tool, a battery may be a battery dedicated to the electrical tool, a battery used as a battery for driving the nursing-care robot 120 in common, or a battery provided separately from a drive battery of the nursing-care robot 120 and used for a plurality of electrical tools in common. When the electrical tool is the type of tool directly connected to the commercial power source and driven, the nursing-care robot control device 30 may make the nursing-care robot 120 perform an operation of inserting and removing a plug on an end portion of a cable of the electrical tool from an electrical socket in the residential room 101 of the care receiver 90.

The ion generator 27A includes a discharge electrode, and applies voltage to the discharge electrode, thereby generating ions as electrically charged microparticles. A type of ions is not particularly limited as long as it contributes to deodorizing or microorganism elimination, thus plasma cluster ion (registered trademark) or nanoe (registered trademark) is appliable, for example. When the nursing-care robot 120 includes the ion generator 27A, the nursing-care robot 120 can generate ions by driving the ion generator 27A in changing the diaper 92. Accordingly, the nursing-care robot 120 can perform odor control or microorganism elimination.

The sprayer 27B includes a tank for storing liquid, a pump, and a nozzle, drives the pump and suctions the liquid in the tank, and sprays the liquid in a misty form from the nozzle. It is applicable that the sprayer 27B includes a manual lever, thus the sprayer 27B itself can drive pump through an operation of the manual lever by the hand 22 and spray the liquid without receiving electrical power supply. It is also applicable that the sprayer 27B does not include a manual lever operated by the hand 22, and the pump is an electrical pump, thus the sprayer 27B itself can receive electrical power supply and spray the liquid, for example. A type of the liquid in the sprayer 27B is optionally set, thus perfume, an air refresher, and an antiseptic agent are applicable, for example. The nursing-care robot 120 may include the plural types of sprayers 27B each including liquid different from each other in the perfume, the air refresher, and the antiseptic agent. When the nursing-care robot 120 includes the sprayer 27B, the nursing-care robot 120 uses the sprayer 27B in changing the diaper 92, thereby being able to generate mist. Accordingly, the nursing-care robot 120 can perform odor control or microorganism elimination.

The air generator 27C includes an electrical compressor and a nozzle, and drives the electrical compressor, thereby spraying air from the nozzle. The air may be cool air or warm air. The cool air and the warm air may be able to be switched. When the nursing-care robot 120 includes the air generator 27C, the nursing-care robot 120 drives the air generator 27C in changing the diaper 92, thereby being able to generate air. For example, the nursing-care robot 120 can apply the generated air to a genital area of the care receiver 90 after bed bath, and dry the genital area.

The electrical vacuum cleaner 27D includes an electrical blower, a vacuum cleaner head, and a dust collection container, and drives the electrical blower, thereby generating suction air from the vacuum cleaner head to the dust collection container and suctioning dust, for example. When the nursing-care robot 120 includes the electrical vacuum cleaner 27D, the nursing-care robot 120 can drive the electrical vacuum cleaner 27D and clean an area around the nursing-care robot 120.

The hand washer 27E includes a tub part for washing the hand 22, a housing part separately housing clean liquid before cleaning and liquid which has been used for cleaning, and a flow path flowing liquid between the housing part and the tub part. A method of washing the hand 22 by the hand washer 27E is optionally set. For example, it is applicable that the hand washer 27E includes an injection part injecting pressurized liquid to the hand 22 to wash the hand 22, a pulsator rotating water in the tub part to wash the hand 22, or an ultrasonic vibrator ultrasonic-vibrating water in the tub part to wash the hand 22. It is also applicable that the hand 22 itself moves and is washed using water flowing from the housing part to the tub part or remaining in the tub part. When the nursing-care robot 120 includes the hand washer 27E, the nursing-care robot 120 can drive the hand washer 27E after changing the diaper 92 and wash the hand 22, for example.

The replacement hand 27F is the hand 22 for being replaced with the hand 22 currently attached to the arm 21. When the nursing-care robot 120 includes the replacement hand 27F, the hand 22 can be replaced. The replacement hand 27F may be the same type of hand 22 as the hand 22 currently attached to the arm 21. In this case, the nursing-care robot 120 may replace the hand 22 with the replacement hand 27F when the hand 22 currently attached to the arm 21 gets dirty. The replacement hand 27F may be a different type of hand 22 from the hand 22 currently attached to the arm 21. In this case, the nursing-care robot 120 may replace the hand 22 with the replacement hand 27F to perform a treatment different from the treatment which the hand 22 currently attached to the arm 21 performs.

When the nursing-care robot 120 includes the garbage box 27G, the nursing-care robot 120 can dispose of a garbage such as the used diaper 92 into the mobile garbage box 27G. The nursing-care robot 120 may dispose of a garbage in the garbage box 27G in the residential room 101 of the care receiver 90 into the garbage box 27G carried by the nursing-care robot 120 itself.

The nursing-care robot 120 includes a stock part 28 for stocking and carrying the mobile tool 27. The stock part 28 stocks some or all of the mobile tools 27. The stock part 28 may stock the supplies such as the diaper 92 and the sheet used to change the diaper 92. The stock part 28 may be integrally formed with the human-like nursing-care robot 120 in an inseparable form. In this case, it is sufficient that the stock part 28 is provided to a position to which the hand 22 of the human-like nursing-care robot 120 can have access. In the example illustrated in Fig. 10, the stock part 28 is provided to be integral with the base-equipped trunk 123 in an inseparable form.

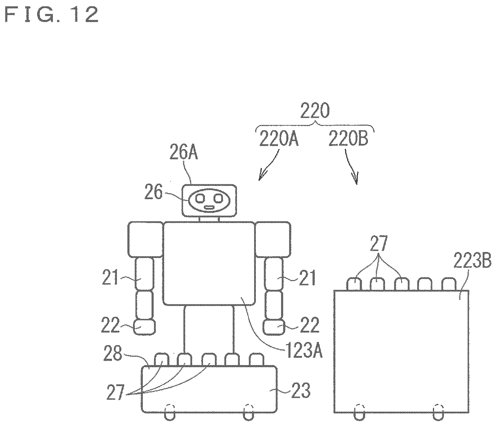

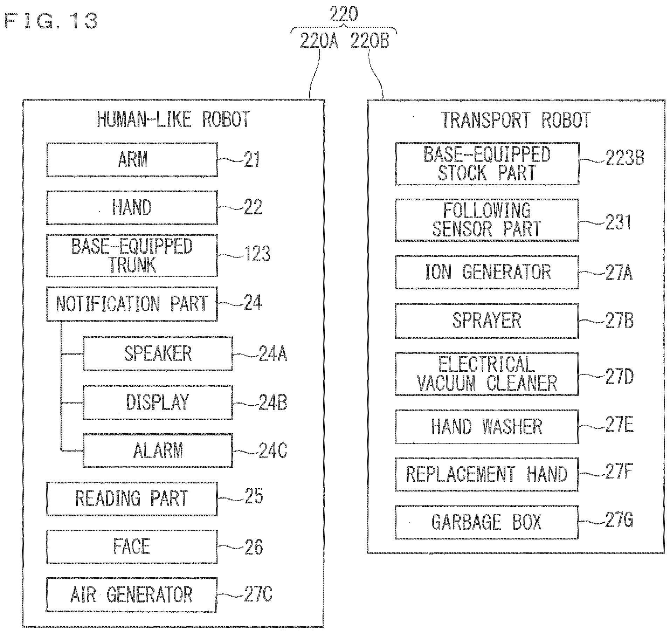

Fig. 12 is a schematic front view illustrating a modification example of the nursing-care robot 120 according to the embodiment 2. Fig. 13 is a functional block diagram illustrating the modification example of the nursing-care robot 120 according to the embodiment 2. As with the example illustrated in Fig. 12, the stock part 28 may be provided to be separable from the human-like nursing-care robot 120. In the example illustrated in Fig. 12, the stock part is also provided to a transport vehicle 220B provided separately from a human-like robot 220A. The transport vehicle 220B includes a base-equipped stock part 223B, and the base-equipped stock part 223B stocks the mobile tool 27. In this case, the human-like robot 220A can appropriately use the mobile tool 27 in the transport vehicle 220B. A configuration of the human-like robot 220A other than the mobile tool 27 can be similar to that of the nursing-care robot 120 described above.

When the human-like robot 220A and the transport vehicle 220B separately carry the plurality of mobile tools 27, a way of dividing the mobile tools 27 is optionally determined. For example, as illustrated in Fig. 13, it is applicable that the human-like robot 220A carries the supplies and the mobile tool 27 used for the treatment on mainly the care receiver 90, and the transport vehicle 220B carries the supplies and the mobile tool 27 used for the treatment on mainly the human-like robot 220A or the residential room 101. When a nursing-care robot 220 goes its rounds of the plurality of care givers, the nursing-care robot 220 carries the plurality of diapers 92 for the plurality of care receivers 90. At this time, it is applicable that the human-like robot 220A carries some diapers 92, and the transport vehicle 220B carries the remaining diapers 92.

It is applicable that the transport vehicle 220B cannot travel by itself, and the human-like robot 220A carries the transport vehicle 220B. In this case, for example, the human-like robot 220A may pull the transport vehicle 220B from a front side or press the transport vehicle 220B from a back side using the hand 22 to move the transport vehicle 220B. For example, it is applicable that a tow part is provided separately from the hand 22, and a towed part towed by the tow part is provided to the transport vehicle 220B. The tow part and the towed part may be detachable.

The transport vehicle 220B can travel by itself. The transport vehicle 220B which can travel by itself can be considered the transport robot 220B. In this case, the nursing-care robot 220 includes the human-like robot 220A changing the diaper 92 and the transport robot 220B provided separately from the human-like robot 220A, and the nursing-care robot control device 30 controls the human-like robot 220A and the transport robot 220B. The nursing-care robot control device 30 may make the transport robot 220B travel by itself to follow the human-like robot 220A. In this case, it is sufficient that a following sensor 231 for following the human-like robot 220A is provided to the transport robot 220B. The nursing-care robot control device 30 may make the human-like robot 220A the transport robot 220B individually travel by themselves.

### <Flow of facility round treatment>

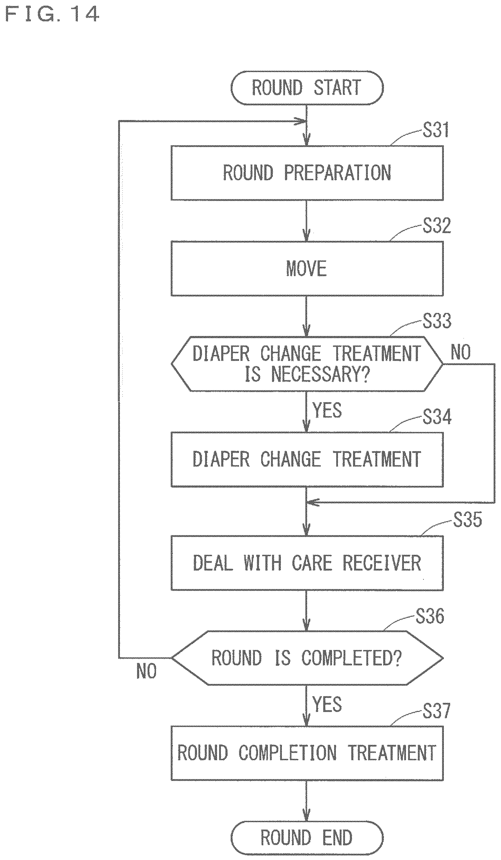

Described is a flow of a treatment of the nursing-care robot 120 going its rounds of the facility 100. Fig. 14 is a flow chart a facility round treatment.

In Step S31, the nursing-care robot control device 30 makes the nursing-care robot 120 prepare for the round. In preparing for the round, the nursing-care robot 120 determines whether the supplies carried in the round are not insufficient, for example. When it is determined that the carried supplies are insufficient, the nursing-care robot 120 may restock the supplies which can be restocked from the supply management station 104 by the nursing-care robot 120 itself. When it is determined that the carried supplies are insufficient, the nursing-care robot 120 may notify a manager to receive restocking from the manager. When the nursing-care robot 120 finishes the preparation for the round, the process proceeds to next Step S32.

In next Step S32, the nursing-care robot control device 30 makes the nursing-care robot 120 move to the next care receiver 90. For example, the storage part 51 stores map data of the facility 100, and the controller 50 can acquire a current position of the nursing-care robot 120 based on the sensor 31, for example. The controller 50 generates a route to the residential room 101 of the care receiver 90 based on the map data and the current position of the nursing-care robot 120, and moves the nursing-care robot 120 along the route. The storage part 51 stores a table indicating an order of the round, and the nursing-care robot control device 30 makes the nursing-care robot 120 move to the earliest care receiver 90 in the order of the round described in the table. The table may be a table in which an order of the residential room 101 for the round is described. In this case, the nursing-care robot 120 may read the ID of the care receiver 90 after reaching the residential room 101, for example.

In next Step S33, the nursing-care robot control device 30 determines whether the diaper 92 of the care receiver 90 needs to be changed. Step S33 is a step, that is determination whether the diaper 92 needs to be changed in Step S1 described above, made to be independent from the pretreatment process. When it is determined that the diaper 92 needs to be changed, the process proceeds to Step S34, and when it is determined that the diaper 92 needs not be changed, the process proceeds to Step S35.

In next step S34, the nursing-care robot 120 changes the diaper 92 of the care receiver 90. The treatment of changing the diaper 92 may be based on Step S1 to Step S7 described above. However, excluded is the determination whether the diaper 92 needs to be changed in the pretreatment in Step S1 described above.

Furthermore, herein, the nursing-care robot control device 30 controls the nursing-care robot 120 so that the nursing-care robot 120 applies air to the care receiver 90 after changing the diaper 92 in Step S34 described above. Herein, the nursing-care robot 120 applies the air to the care receiver 90 after changing the diaper 92 before Step S6 after Step S2 performed in Step S34.

Furthermore, herein, the nursing-care robot control device 30 controls the nursing-care robot 120 so that the nursing-care robot 120 performs the odor control treatment in Step S34 described above. For example, the odor control treatment may be air ventilation of the residential room 101 of the care receiver 90 by opening a window or activating a ventilation fan. For example, the odor control treatment may be generation of ions by driving the ion generator 27A. For example, the odor control treatment may be spraying perfume or an air refresher using the sprayer 27B. Herein, the nursing-care robot 120 performs the odor control treatment in a period of time when Step S1 is performed until Step S7 is completed in Step S34. For example, it is sufficient that air ventilation and ion generation are performed in the pretreatment in Step S1. For example, it is sufficient that spraying perfume or an air refresher is performed in the posttreatment in Step S7.

Furthermore, herein, the nursing-care robot control device 30 controls the nursing-care robot 120 so that the nursing-care robot 120 cleans an area around the care receiver 90 in Step S34 described above. At the time of the posttreatment in Step S7 performed in Step S34, the nursing-care robot 120 performs an environment cleaning operation of cleaning an environment such as the care receiver 90 after the diaper 92 is changed and an area around the bed 95 of the care receiver 90. For example, the environment cleaning operation may be operating the electrical vacuum cleaner 27D. For example, the environment cleaning operation may be gripping a wiping member (wet tissue, for example) by the hand to perform wiping and cleaning. For example, the environment cleaning operation may be spraying an antiseptic agent using the sprayer 27B. For example, the environment cleaning operation may be driving the ion generator 27A to generate ions.

Furthermore, herein, the nursing-care robot control device 30 controls the nursing-care robot 120 so that the nursing-care robot 120 cleans the hand 22 in Step S34 described above. The nursing-care robot 120 performs a hand cleaning operation of cleaning the hand 22 by the nursing-care robot 120 itself in the posttreatment in Step S7 performed in Step S34. The hand cleaning operation may be washing the hand 22, for example. For example, the hand cleaning operation may be dry-wiping or wet-wiping the hand 22. For example, the hand cleaning operation may be eliminating microorganism.

The nursing-care robot 120 may perform a hand replacement operation of replacing the hand 22 attached to the arm 21 with the replacement hand 27F which is not attached to the arm 21 by the nursing-care robot 120 itself. The nursing-care robot 120 may perform the hand cleaning operation on the hand 22 which has been replaced and detached. It is also applicable that a hand housing part is provided to the base 23 and the hand 22 can be detached from the arm 21 while the hand 22 on the tip end of the arm 21 is housed in the hand housing part.

The nursing-care robot 120 may perform an operation of attaching and detaching a glove for the robot hand 22 by the nursing-care robot 120 itself. The nursing-care robot 120 may include a space to house the glove for replacement. The glove for the robot hand 22 may be disposable. In this case, the nursing-care robot 120 may dispose of a used glove after replacement into the garbage box 27G. The glove for the robot hand 22 may be repetitively used. In this case, the nursing-care robot 120 may house the used glove after replacement separately from the garbage box 27G. The nursing-care robot 120 may perform a cleaning operation on the glove for the robot hand 22 which has been replaced and detached.

In next step S35, the nursing-care robot 120 deals with the care receiver 90. Dealing with the care receiver 90 includes communication with the care receiver 90. Dealing with the care receiver 90 may be performed in parallel to a part of the posttreatment in Step S7 performed in Step S34 described above.

In next step S36, the nursing-care robot 120 determines whether the round is completed. When the nursing-care robot 120 has not gone its rounds of all of the scheduled care receivers 90 described in the table, the process is returned to Step S31, and the nursing-care robot 120 goes its rounds of the care receiver 90 described in the table next to the care receiver 90 which has been done this time. When the nursing-care robot 120 has gone its rounds of all of the scheduled care receivers 90 described in the table, it is determined that the round is completed, and the process proceeds to Step S37.

A round completion treatment is performed in Step S37. For example, the round completion treatment is transferring a garbage in the garbage box 27G to the garbage pickup station 105. Subsequently, the nursing-care robot 120 returns to the robot station 102. In the robot station 102, the nursing-care robot 120 restocks the supplies from the supply management station 104, or has access to the power supply part 103 to receive electrical power supply.

### <Effect etc.>

Also according to the nursing-care robot system 110 having the above configuration, the nursing-care robot 120 can be made to change the diaper 92 in accordance with the body type and the posture of the care receiver 90 in the manner similar to the nursing-care robot 20 described above. Accordingly, the nursing-care robot system 110 can perform the delicate nursing-care behavior such as change of the diaper 92 in place of a person. Furthermore, when the nursing-care robot 120 has the human-like shape, the care receiver 90 hardly becomes anxious about the treatment of changing the diaper 92 by the nursing-care robot 120.

The elimination treatment program is incorporated into a round program so that the nursing-care robot 120 executes the elimination treatment program when the nursing-care robot 120 goes its rounds of the facility 100 and visits to each of the plurality of care receivers 90. Accordingly, the nursing-care robot 120 can change the diaper 92 of each of the plurality of care givers in the facility 100 in sequence.

The nursing-care robot control device 30 controls the nursing-care robot 120 so that the nursing-care robot 120 carries the garbage box 27G and disposes of the garbage in changing the diaper 92 into the garbage box 27G. Accordingly, time and effort of a staff in the facility 100 picking up the garbage from the residential room 101 of the care receiver 90 can be suppressed.

The nursing-care robot control device 30 controls the nursing-care robot 120 so that the nursing-care robot 120 disposes of the garbage in the garbage box 27G into the garbage pickup station 105 disposed in the facility 100. Accordingly, time and effort of a staff in the facility 100 picking up the garbage from the nursing-care robot 120 can be suppressed.

The nursing-care robot control device 30 determines whether the diaper 92 of the care receiver 90 needs to be changed before the nursing-care robot 120 changes the diaper 92 of the care receiver 90. The nursing-care robot control device 30 generates the elimination treatment instruction when determining that the diaper 92 needs to be changed, and controls the nursing-care robot 120 so that the nursing-care robot 120 does not change the diaper 92 when determining that the diaper 92 needs not be changed. Accordingly, change of the diaper 92 can be suppressed in the case where the diaper 92 needs not be changed.

The nursing-care robot control device 30 controls the nursing-care robot 120 so that the nursing-care robot 120 has communication with the care receiver 90. Accordingly, improvement of life or mental care of the care receiver 90 can be easily achieved.

Furthermore, herein, the nursing-care robot control device 30 controls the nursing-care robot 120 so that the nursing-care robot 120 performs the odor control treatment. Accordingly, the care receiver 90 or a person around the care receiver 90 hardly smells the odor in accordance with change of the diaper 92.

When the nursing-care robot 120 detects abnormality of the nursing-care robot 120 itself, the nursing-care robot control device 30 controls the nursing-care robot 120 so that the nursing-care robot 120 transmits notification to a manager. Accordingly, the manager easily detects the abnormality of the nursing-care robot 120.

The nursing-care robot control device 30 controls the nursing-care robot 120 so that the nursing-care robot 120 transmits notification to a manager when the nursing-care robot 120 detects abnormality of a surrounding environment. Accordingly, the manager easily detects the abnormality of the surrounding environment of the nursing-care robot 120.

The nursing-care robot control device 30 controls the nursing-care robot 120 so that the nursing-care robot 120 applies air to the care receiver 90 after changing the diaper 92. Accordingly, a genital area of the care receiver 90 can be dried after the diaper 92 is changed.

The nursing-care robot control device 30 controls the nursing-care robot 120 so that the nursing-care robot 120 cleans an area around the care receiver 90. Accordingly, the area around the care receiver 90 can be kept clean by the nursing-care robot 120.

The nursing-care robot control device 30 controls the nursing-care robot 120 so that the nursing-care robot 120 cleans the hand 22. Accordingly, the hand 22 can be kept clean by the nursing-care robot 120 itself.

The nursing-care robot control device 30 controls the nursing-care robot 120 so that the nursing-care robot 120 replaces the hand 22. Accordingly, replacement of the hand 22 by a person can be suppressed.

### <Appendix>

Described above is the example that the nursing-care robot system 10 performs the elimination treatment as the nursing-care behavior, however, the nursing-care robot system 10 may perform the nursing-care behavior other than the elimination treatment. For example, the nursing-care robot system may make the care receiver 90 take a bath. The example of changing the diaper 92 with the tape 93 is described as the elimination treatment, however, the nursing-care robot system 10 may perform the elimination treatment other than change of the diaper 92 with the tape 93. For example, the nursing-care robot system may change an underpants-type diaper. The nursing-care robot system may perform the elimination treatment of setting a vacuum to a genital area.

Each configuration described in each embodiment and modification example described above can be appropriately combined as long as they are not contradictory.

The present specification and the drawings disclose each aspect described hereinafter.

A first aspect is a nursing-care robot system including: a nursing-care robot control device; and a human-like nursing-care robot performing a nursing-care behavior controlled by the nursing-care robot control device, wherein the nursing-care robot control device includes a storage part storing a behavior program, a state variable acquisition part acquiring a state variable corresponding to a state of a care receiver, and an instruction generation part generating a behavior instruction to the nursing-care robot from the behavior program and the state variable, the behavior program includes an elimination treatment program for the nursing-care robot to perform an elimination treatment for changing a diaper of the care receiver, the instruction generation part generates an elimination treatment instruction corresponding to the care receiver from the elimination treatment program as the behavior instruction, the state variable includes a state variable, which is a state variable used for adjusting the elimination treatment instruction, corresponding to positional data, a body type, and a posture of the care receiver, and the nursing-care robot includes a trunk with a base which can travel by itself, an arm extending from the trunk, a hand provided to the arm, and a face.

According to the first aspect, the nursing-care robot can be made to change a diaper in accordance with a body type and a posture of the care receiver. Accordingly, the nursing-care robot system can perform a delicate nursing-care behavior such as change of the diaper in place of a person. The nursing-care robot has the human-like shape, thus the care receiver hardly becomes anxious about the treatment of changing the diaper.

A second aspect is the nursing-care robot system according to the first aspect, wherein the behavior program includes a round program of making the nursing-care robot travel by itself and go its rounds of a facility admitting a plurality of care receivers, and the elimination treatment program is incorporated into the round program so that the nursing-care robot executes the elimination treatment program when the nursing-care robot visits to each of the plurality of care receivers. Accordingly, the nursing-care robot can change the diaper of the plurality of care givers in the facility in sequence.

A third aspect is the nursing-care robot system according to the second aspect, wherein the nursing-care robot control device controls the nursing-care robot so that the nursing-care robot carries a garbage box and disposes of a garbage in changing the diaper into the garbage box. Accordingly, time and effort of a staff in the facility picking up the garbage from a residential room of the care receiver can be suppressed, for example.

A fourth aspect is the nursing-care robot system according to the third aspect, wherein the nursing-care robot control device controls the nursing-care robot so that the nursing-care robot disposes of a garbage in the garbage box into a garbage pickup station disposed in the facility. Accordingly, time and effort of a staff in the facility picking up the garbage from the nursing-care robot can be suppressed, for example.

A fifth aspect is the nursing-care robot system according to any one of the first to fourth aspects, wherein the nursing-care robot control device determines whether the diaper of the care receiver needs to be changed before the nursing-care robot changes the diaper of the care receiver, generates the elimination treatment instruction when determining that the diaper needs to be changed, and controls the nursing-care robot so that the nursing-care robot does not change the diaper of the care receiver when determining that the diaper needs not be changed. Accordingly, change of the diaper can be suppressed in the case where the diaper needs not be changed.

A sixth aspect is the nursing-care robot system according to any one of the first to fifth aspects, wherein the nursing-care robot control device controls the nursing-care robot so that the nursing-care robot has communication with the care receiver. Accordingly, improvement of life or mental care of the care receiver can be easily achieved.

A seventh aspect is the nursing-care robot system according to any one of the first to sixth aspects, wherein the nursing-care robot control device controls the nursing-care robot so that the nursing-care robot performs an odor control treatment. Accordingly, the care receiver or a person around the care receiver hardly smells odor in accordance with change of the diaper.

An eighth aspect is the nursing-care robot system according to any one of the first to seventh aspects, wherein the nursing-care robot control device controls the nursing-care robot so that the nursing-care robot transmits notification to a manager when the nursing-care robot detects abnormality of the nursing-care robot itself. Accordingly, the manager easily detects the abnormality of the nursing-care robot.

A ninth aspect is the nursing-care robot system according to any one of the first to eighth aspects, wherein the nursing-care robot control device controls the nursing-care robot so that the nursing-care robot transmits notification to a manager when the nursing-care robot detects abnormality of a surrounding environment. Accordingly, the manager easily detects the abnormality around the nursing-care robot.

A tenth aspect is the nursing-care robot system according to any one of the first to ninth aspects, wherein the nursing-care robot control device controls the nursing-care robot so that the nursing-care robot applies air to the care receiver after the diaper is changed. Accordingly, a genital area of the care receiver can be dried after the diaper is changed.

An eleventh aspect is the nursing-care robot system according to any one of the first to tenth aspects, wherein the nursing-care robot control device controls the nursing-care robot so that the nursing-care robot cleans an area around the care receiver. Accordingly, the area around the care receiver can be kept clean by the nursing-care robot.

A twelfth aspect is the nursing-care robot system according to any one of the first to eleventh aspects, wherein the nursing-care robot control device controls the nursing-care robot so that the nursing-care robot cleans the hand. Accordingly, the hand can be kept clean by the nursing-care robot itself.

A thirteenth aspect is the nursing-care robot system according to any one of the first to twelfth aspects, wherein the nursing-care robot control device controls the nursing-care robot so that the nursing-care robot replaces the hand. Accordingly, replacement of the hand 22 by a person can be suppressed.

A fourteenth aspect is a nursing-care robot control device controlling a nursing-care robot performing a nursing-care behavior, including: a storage part storing a behavior program; a state variable acquisition part acquiring a state variable corresponding to a state of a care receiver; and an instruction generation part generating a behavior instruction to the nursing-care robot from the behavior program and the state variable, wherein the behavior program includes an elimination treatment program for the nursing-care robot to perform an elimination treatment for changing a diaper of the care receiver, the instruction generation part generates an elimination treatment instruction corresponding to the care receiver from the elimination treatment program as the behavior instruction, the state variable includes a first state variable, which is a state variable used for adjusting the elimination treatment instruction, corresponding to positional data, a body type, and a posture of the care receiver and a second state variable corresponding to sex of the care receiver, and the second state variable is used for adjusting the elimination treatment instruction corresponding to a position of attaching a urine absorption pad, a method of cleaning a genital area, or a method of performing bed bath on a genital area. According to the fourteenth aspect, the nursing-care robot can be made to perform the elimination treatment in accordance with a body type and a posture of the care receiver. Accordingly, the nursing-care robot system can perform a delicate nursing-care behavior such as change of the diaper in place of a person. The nursing-care robot system can provide more appropriate nursing care in accordance with the sex of the care receiver.

A fifteenth aspect is a nursing-care robot control device controlling a nursing-care robot performing a nursing-care behavior, including: a storage part storing a behavior program; a state variable acquisition part acquiring a state variable corresponding to a state of a care receiver; and an instruction generation part generating a behavior instruction to the nursing-care robot from the behavior program and the state variable, wherein the behavior program includes an elimination treatment program for the nursing-care robot to perform an elimination treatment for changing a diaper of the care receiver, the instruction generation part generates an elimination treatment instruction corresponding to the care receiver from the elimination treatment program as the behavior instruction, the state variable includes a first state variable, which is a state variable used for adjusting the elimination treatment instruction, corresponding to positional data, a body type, and a posture of the care receiver, and the first state variable corresponding to the body type of the care receiver is set based on ID information read by a reading part identifying ID information of the care receiver. According to the fifteenth aspect, the nursing-care robot can be made to perform the elimination treatment in accordance with a body type and a posture of the care receiver. Accordingly, the nursing-care robot system can perform a delicate nursing-care behavior such as change of the diaper in place of a person. The first state variable corresponding to the body type of the care receiver can be easily acquired without a taken image of the care receiver.

A sixteenth aspect is a nursing-care robot control device controlling a nursing-care robot performing a nursing-care behavior, including: a storage part storing a behavior program; a state variable acquisition part acquiring a state variable corresponding to a state of a care receiver; and an instruction generation part generating a behavior instruction to the nursing-care robot from the behavior program and the state variable, wherein the behavior program includes an elimination treatment program for the nursing-care robot to perform an elimination treatment for changing a diaper of the care receiver, the instruction generation part generates an elimination treatment instruction corresponding to the care receiver from the elimination treatment program as the behavior instruction, the state variable includes a first state variable, which is a state variable used for adjusting the elimination treatment instruction, corresponding to positional data, a body type, and a posture of the care receiver, and it is determined whether the elimination treatment needs to be performed based on a detection result of change of a color or a degree of wetness of the diaper, and the elimination treatment instruction is generated when it is determined that the elimination treatment needs to be performed. According to the sixteenth aspect, the nursing-care robot can be made to perform the elimination treatment in accordance with a body type and a posture of the care receiver. Accordingly, the nursing-care robot system can perform a delicate nursing-care behavior such as change of the diaper in place of a person. The nursing-care robot can be made to change the diaper when the diaper needs to be changed.

A seventeenth aspect is a nursing-care robot control device according to any one of the fourteenth to sixteenth aspects, wherein the elimination treatment program is a program mechanically learned to imitate a model nursing-care behavior based on model nursing-care data in the model nursing-care behavior, and the model nursing-care data includes coordinate data of a nursing-care hand and outline data of a model care receiver in the model nursing-care behavior. Accordingly, accuracy of the elimination treatment program is improved.

An eighteenth aspect is a nursing-care robot control device according to any one of the fourteenth to seventeenth aspects, wherein the model nursing-care data includes data of at least one of an acceleration sensor and/or a pressure sensor attached to the nursing-care hand. Accordingly, accuracy of degree of force in the nursing-care robot is improved.

A nineteenth aspect is the nursing-care robot control device according to any one of the fourteenth to eighteenth aspects, wherein the state variable includes a third state variable, which is a state variable used for adjusting the elimination treatment instruction, corresponding to feeling of the care receiver in the elimination treatment behavior of the nursing-care robot. Accordingly, the nursing-care robot system can provide more appropriate nursing care in accordance with the feeling of the care receiver.

A twentieth aspect is the nursing-care robot control device according to any one of the fourteenth to nineteenth aspects, further including an additional learning part outputting the elimination treatment program updated based on the state variable acquired by the state variable acquisition part in the elimination treatment behavior of the nursing-care robot on the care receiver. Accordingly, accuracy of the elimination treatment program is improved more as the elimination treatment behavior is repetitively performed by the nursing-care robot.

A twenty-first aspect is a nursing-care robot system, including: the nursing care robot control device according to any one of the fourteenth to the twentieth aspects; and a nursing-care robot performing a nursing-care behavior controlled by the nursing-care robot control device. According to the present nursing-care robot system, the nursing-care robot is controlled by the nursing-care robot control device described above, thus can be made to change the diaper in accordance with the body type and the posture of the care receiver. Accordingly, the nursing-care robot system can perform a delicate nursing-care behavior such as change of the diaper in place of a person.

The foregoing description is in all aspects illustrative, thus the present invention is not limited thereto. It is therefore understood that numerous modification examples can be devised without departing from the scope of the invention.

### EXPLANATION OF REFERENCE SIGNS

10, 110 nursing-care robot system
20, 120, 220 nursing-care robot
21 arm
22 hand
23 base
26 face
27 mobile tool
27A ion generator
27B sprayer
27C air generator
27D electrical vacuum cleaner
27E hand washer
27F replacement hand
27G garbage box
30 nursing-care robot control device
31 sensor
32 surrounding environment detection sensor
33, 33A, 33B camera
34 microphone
35 humidity sensor
36 pulsation sensor
40 robot operation detection sensor
41 pressure sensor
42 acceleration sensor
43 angular sensor
50 controller
51 storage part
51a program
52 processor
53 communication part
70 imitative learning device
75 camera
76 positional sensor
77 pressure sensor
78 acceleration sensor
80 additional learning part
90 care receiver
91 clothing
92 diaper
93 tape
100 facility
10 residential room
102 robot station
105 garbage pickup station
123 base-equipped trunk
123A trunk

## Claims

1. A nursing-care robot system, comprising:
a nursing-care robot control device; and
a human-like nursing-care robot performing a nursing-care behavior controlled by the nursing-care robot control device, wherein
the nursing-care robot control device includes a storage part storing a behavior program, a state variable acquisition part acquiring a state variable corresponding to a state of a care receiver, and an instruction generation part generating a behavior instruction to the nursing-care robot from the behavior program and the state variable,
the behavior program includes an elimination treatment program for the nursing-care robot to perform an elimination treatment for changing a diaper of the care receiver,
the instruction generation part generates an elimination treatment instruction corresponding to the care receiver from the elimination treatment program as the behavior instruction,
the state variable includes a state variable, which is a state variable used for adjusting the elimination treatment instruction, corresponding to positional data, a body type, and a posture of the care receiver, and
the nursing-care robot includes a trunk with a base which can travel by itself, an arm extending from the trunk, a hand provided to the arm, and a face.

2. The nursing-care robot system according to claim 1, wherein
the behavior program includes a round program of making the nursing-care robot travel by itself and go its rounds of a facility admitting a plurality of care receivers, and
the elimination treatment program is incorporated into the round program so that the nursing-care robot executes the elimination treatment program when the nursing-care robot visits to each of the plurality of care receivers.

3. The nursing-care robot system according to claim 2, wherein
the nursing-care robot control device controls the nursing-care robot so that the nursing-care robot carries a garbage box and disposes of a garbage in changing the diaper into the garbage box.

4. The nursing-care robot system according to claim 3, wherein
the nursing-care robot control device controls the nursing-care robot so that the nursing-care robot disposes of a garbage in the garbage box into a garbage pickup station disposed in the facility.

5. The nursing-care robot system according to any one of claims 1 to 4, wherein
the nursing-care robot control device determines whether the diaper of the care receiver needs to be changed before the nursing-care robot changes the diaper of the care receiver, generates the elimination treatment instruction when determining that the diaper needs to be changed, and controls the nursing-care robot so that the nursing-care robot does not change the diaper of the care receiver when determining that the diaper needs not be changed.

6. The nursing-care robot system according to any one of claims 1 to 4, wherein
the nursing-care robot control device controls the nursing-care robot so that the nursing-care robot has communication with the care receiver.

7. The nursing-care robot system according to any one of claims 1 to 4, wherein
the nursing-care robot control device controls the nursing-care robot so that the nursing-care robot performs an odor control treatment.

8. The nursing-care robot system according to any one of claims 1 to 4, wherein
the nursing-care robot control device controls the nursing-care robot so that the nursing-care robot transmits notification to a manager when the nursing-care robot detects abnormality of the nursing-care robot itself.

9. The nursing-care robot system according to any one of claims 1 to 4, wherein
the nursing-care robot control device controls the nursing-care robot so that the nursing-care robot transmits notification to a manager when the nursing-care robot detects abnormality of a surrounding environment.

10. The nursing-care robot system according to any one of claims 1 to 4, wherein
the nursing-care robot control device controls the nursing-care robot so that the nursing-care robot applies air to the care receiver after the diaper is changed.

11. The nursing-care robot system according to any one of claims 1 to 4, wherein
the nursing-care robot control device controls the nursing-care robot so that the nursing-care robot cleans an area around the care receiver.

12. The nursing-care robot system according to any one of claims 1 to 4, wherein
the nursing-care robot control device controls the nursing-care robot so that the nursing-care robot cleans the hand.

13. The nursing-care robot system according to any one of claims 1 to 4, wherein
the nursing-care robot control device controls the nursing-care robot so that the nursing-care robot replaces the hand.

14. A nursing-care robot control device controlling a nursing-care robot performing a nursing-care behavior, comprising:
a storage part storing a behavior program;
a state variable acquisition part acquiring a state variable corresponding to a state of a care receiver; and
an instruction generation part generating a behavior instruction to the nursing-care robot from the behavior program and the state variable, wherein
the behavior program includes an elimination treatment program for the nursing-care robot to perform an elimination treatment for changing a diaper of the care receiver,
the instruction generation part generates an elimination treatment instruction corresponding to the care receiver from the elimination treatment program as the behavior instruction,
the state variable includes a first state variable, which is a state variable used for adjusting the elimination treatment instruction, corresponding to positional data, a body type, and a posture of the care receiver and a second state variable corresponding to sex of the care receiver, and
the second state variable is used for adjusting the elimination treatment instruction corresponding to a position of attaching a urine absorption pad, a method of cleaning a genital area, or a method of performing bed bath on a genital area.

15. A nursing-care robot control device controlling a nursing-care robot performing a nursing-care behavior, comprising:
a storage part storing a behavior program;
a state variable acquisition part acquiring a state variable corresponding to a state of a care receiver; and
an instruction generation part generating a behavior instruction to the nursing-care robot from the behavior program and the state variable, wherein
the behavior program includes an elimination treatment program for the nursing-care robot to perform an elimination treatment for changing a diaper of the care receiver,
the instruction generation part generates an elimination treatment instruction corresponding to the care receiver from the elimination treatment program as the behavior instruction,
the state variable includes a first state variable, which is a state variable used for adjusting the elimination treatment instruction, corresponding to positional data, a body type, and a posture of the care receiver, and
the first state variable corresponding to the body type of the care receiver is set based on ID information read by a reading part identifying ID information of the care receiver.

16. A nursing-care robot control device controlling a nursing-care robot performing a nursing-care behavior, comprising:
a storage part storing a behavior program;
a state variable acquisition part acquiring a state variable corresponding to a state of a care receiver; and
an instruction generation part generating a behavior instruction to the nursing-care robot from the behavior program and the state variable, wherein
the behavior program includes an elimination treatment program for the nursing-care robot to perform an elimination treatment for changing a diaper of the care receiver,
the instruction generation part generates an elimination treatment instruction corresponding to the care receiver from the elimination treatment program as the behavior instruction,
the state variable includes a first state variable, which is a state variable used for adjusting the elimination treatment instruction, corresponding to positional data, a body type, and a posture of the care receiver, and
it is determined whether the elimination treatment needs to be performed based on a detection result of change of a color or a degree of wetness of the diaper, and the elimination treatment instruction is generated when it is determined that the elimination treatment needs to be performed.

17. The nursing-care robot control device according to any one of claims 14 to 16, wherein
the elimination treatment program is a program mechanically learned to imitate a model nursing-care behavior based on model nursing-care data in the model nursing-care behavior, and
the model nursing-care data includes coordinate data of a nursing-care hand and outline data of a model care receiver in the model nursing-care behavior.

18. The nursing-care robot control device according to claim 17, wherein
the model nursing-care data includes data of at least one of an acceleration sensor and/or a pressure sensor attached to the nursing-care hand.

19. The nursing-care robot control device according to any one of claims 14 to 16, wherein
the state variable includes a third state variable, which is a state variable used for adjusting the elimination treatment instruction, corresponding to feeling of the care receiver in the elimination treatment behavior of the nursing-care robot.

20. The nursing-care robot control device according to any one of claims 14 to 16, further comprising
an additional learning part outputting the elimination treatment program updated based on the state variable acquired by the state variable acquisition part in the elimination treatment behavior of the nursing-care robot on the care receiver.

21. A nursing-care robot system, comprising:
the nursing care robot control device according to any one of claims 14 to 16; and
a nursing-care robot performing a nursing-care behavior controlled by the nursing-care robot control device.
